# EUROPÄISCHE PATENTANMELDUNG

(11) **EP 2 759 766 A2**
(43) Veröffentlichungstag der Anmeldung: **30.07.2014**
(21) Anmeldenummer: 14000121.5
(22) Anmeldetag: 14.01.2014
(51) Int. Cl.: F22B 37/56, F23G 5/48, F23G 5/50, F23J 3/02, F23N 5/00, G01N 1/22, G01N 1/10

(54) **Messapparatur, Verfahren zum Untersuchen von Belägen auf einer Belagssonde, Verbrennungsanlage und Verfahren zum Betrieb einer derartigen Verbrennungsanlage**

(30) Priorität: 28.01.2013 DE 102013001361; 04.07.2013 DE 102013011149
(71) Anmelder: MARTIN GmbH für Umwelt- und Energietechnik, 80807 München (DE)
(72) Erfinder: Beckmann, Michael, DE - 01187 Dresden (DE); Rostowski, Slawomir, DE - 01097 Dresden (DE); Spiegel, Wolfgang, DE - 86415 Mering (DE); von Raven, Robert, DE - 82402 Seeshaupt (DE)
(74) Vertreter: Patentanwaltskanzlei Liermann-Castell & Kollegen

(57) **Zusammenfassung**

Die Erfindung betrifft eine Messapparatur mit einer Belagssonde (4) zur Analyse der Ablagerungen in einer Verbrennungsanlage. Ein Sondenhalter (8) ist an einer Wand (7) eines auf über 100° C beheizten Proberaumes (6) angeordnet. Die Belagssonde (4) ist an einer Seite (9) in den Proberaum (6) und an einer gegenüberliegenden Seite (10) aus dem Proberaum (6) heraus in den Innenraum (11) der Verbrennungsanlage verschiebbar. Der Proberaum (6) kann ein Messfenster (14), einen Tropfengeber (16), ein Sichtfenster, und zwei gegenüberliegende abdichtbare Belagssondendurchlässe aufweisen. Die Belagssonde (4) kann zur Beeinflussung ihrer Temperatur Fluidleitungen und Temperaturmesseinrichtungen aufweisen. Ein Reinigungsgerät der Verbrennungsanlage (4) ist über eine Kontrolleinheit mit der Messapparatur (4) gekoppelt.

## Beschreibung

Die Erfindung betrifft eine Messapparatur, ein Verfahren zum Untersuchen von Belägen auf einer Belagssonde, eine Verbrennungsanlage, die mit Verbrennungsgasen durchströmbar ist, und ein Verfahren zum Betrieb einer derartigen Verbrennungsanlage.

Insbesondere betrifft die Erfindung eine Messapparatur mit einer Belagssonde und einem Sondenhalter und ein Verfahren zum Untersuchen von Belägen auf einer Belagssonde, bei dem die Belagssonde in einer Verbrennungsanlage angeordnet wird.

In Verbrennungsanlagen entstehen insbesondere beim Einsatz fester Brennstoffe Beläge an den Wärmeübertragungsflächen. Diese Beläge behindern den Wärmetransport vom Rauchgas der festen Brennstoffe an das Arbeitsmedium. Die in den Belägen enthaltenen Stoffe können aber auch Korrosionsprozesse an der Oberfläche der Kesselwand oder an Wärmetauscherrohren fördern. Letztlich kommt es in Folge der isolierenden Wirkung der Beläge an den Strahlungszügen größerer Verbrennungsanlagen zur Verschiebung des Temperaturprofils entlang des Rauchgasweges, sodass nachgeschaltete Wärmeübertragungsflächen einer erhöhten thermischen Belastung ausgesetzt sind.

Um diesen negativen Auswirkungen der Belagsbildung vorzubeugen, werden die Beläge mit Hilfe von Reinigungssystemen, vorzugsweise während des Betriebs der Feuerungsanlage, entfernt.

Um die Wirkung eines Reinigungssystems auf derartige Belagsschichten zu überprüfen, wird eine Messapparatur mit einer Belagssonde und einem Sondenhalter eingesetzt. Der Sondenhalter ermöglicht es, eine Sonde in einer Feuerungsanlage zu positionieren. Dazu wird die Belagssonde durch eine Öffnung in der Kesselwand in den Rauchgasbereich eingeführt und über den Sondenhalter an der Kesselwand gehalten. Nachdem die Belagssonde eine definierte Zeit den Rauchgasen ausgesetzt worden ist, kann sie aus dem Rauchgasweg entnommen werden, sodass die auf der Sonde anhaftenden Beläge untersucht werden können. Derartige Belagsuntersuchungen sind sinnvoll, um den Zeitpunkt der Reinigung zu bestimmen und die Art des Reinigungsverfahrens festzulegen. Beispielsweise können über Düsen Wassertropfen auf die Beläge gesprüht werden, die auf den Belägen derart verdampfen sollen, dass sie ein Abplatzen des Belages bewirken.

Es hat sich jedoch herausgestellt, dass ein im Labor durchgeführtes Reinigungsverfahren zur Entfernung von Belägen auf der Belagssonde nicht zwangsläufig auch zu entsprechend positiven Ergebnissen bei der Belagsentfernung in der Praxis führt.

Der Erfindung liegt daher die Aufgabe zu Grunde, eine gattungsgemäße Messapparatur mit einer Belagssonde und einem Sondenhalter weiter zu entwickeln, ein verbessertes Verfahren zum Untersuchen von Belägen auf einer Belagssonde, eine Verbrennungsanlage und ein Verfahren zum Betrieb einer derartigen Verbrennungsanlage bereitzustellen.

Diese Aufgabe wird vorrichtungsmäßig mit einer gattungsgemäßen Messapparatur gelöst, bei der der Sondenhalter an einer Wand eines Proberaumes angeordnet ist und die Belagssonde an einer Seite in den Proberaum und an einer gegenüberliegenden Seite aus dem Proberaum heraus verschiebbar ist.

Ein derartiger Proberaum kann an der Wand einer Feuerungsanlage angeordnet werden und er erlaubt es, die Belagssonde aus dem Feuerraum in den Proberaum zu ziehen und im Proberaum zu untersuchen. Der Proberaum ermöglicht es, die atmosphärischen Bedingungen, wie insbesondere die Temperatur, an die Temperatur im Feuerraum anzupassen und die Untersuchungen bei feuerraumähnlichen Umgebungsbedingungen durchzuführen, ohne dass die Belagssonde nach der Entnahme aus dem Feuerraum kälteren Gasen und insbesondere Luftfeuchtigkeit ausgesetzt wird.

Um im Proberaum feuerraumähnliche Bedingungen zu erzielen, können Rauchgase aus dem Feuerraum durch den Proberaum strömen. Um eine Abkühlung der Gase in Proberaum zu vermeiden wird vorgeschlagen, dass der Proberaum beheizt ist.

Außerdem kann die Abkühlung dadurch verringert werden, dass der Proberaum wärmeisoliert ist.

Im Proberaum kann die Belagssonde unterschiedlichen Messeinrichtungen ausgesetzt werden. Dabei kann mechanisch die Festigkeit des Belags geprüft werden. Mit chemischen Methoden kann die Zusammensetzung der Beläge ermittelt werden und Ultraschall- oder Strahlungsverfahren können Informationen über die Art der Beläge bereitstellen. Da die hierfür notwendigen Messeinrichtungen den im Proberaum herrschenden atmosphärischen Bedingungen nicht ausgesetzt werden sollten, wird vorgeschlagen, dass der Proberaum ein Messfenster aufweist.

Für einfache optische Untersuchungen wird vorgeschlagen, dass der Proberaum ein Sichtfenster aufweist.

Um zu untersuchen, wie ein Belag auf einen auftreffenden Wassertropfen reagiert, wird vorgeschlagen, dass der Proberaum einen Tropfengeber aufweist. Dieser Tropfengeber kann einzelne Tropfen unterschiedlicher Größe auf die Sonde fallen lassen. Er kann jedoch auch eine Sprüheinrichtung wie eine Düse aufweisen, über die Wassertropfen oder Chemikalien auf die Belagssonde gegeben werden.

Eine vorteilhafte Ausführungsvariante sieht vor, dass der Proberaum zwei gegenüberliegende abdichtbare Belagssondendurchlässe aufweist. Dies ermöglicht es, die üblicherweise lange Sonde durch den Proberaum zu ziehen und nach Entnahme der Sonde aus dem Kesselraum den zum Kesselraum weisenden Belagssondendurchlass zu verschließen. Dadurch kann der Proberaum vom Feuerraum getrennt werden, während sich die Belagssonde im Proberaum befindet.

Dies ermöglicht es den Proberaum in einem Labor zu untersuchen und die Messungen auch weit entfernt von der Feuerungsanlage durchzuführen. Die Beheizung des Proberaums dient dann dazu, während des Transports und der Untersuchungen feuerraumartige Bedingungen zu erhalten, sodass der Belag auf der Messsonde durch die ihn umgebende Atmosphäre nicht verändert wird.

Um realistische Bedingungen an der Belagssonde zu schaffen, wird vorgeschlagen, dass die Belagssonde Fluidleitungen zur Beeinflussung der Sondentemperatur aufweist. Dadurch kann die Belagssonde über Heißluft, Wasser, Öl etc. derart temperiert werden, dass die Oberflächentemperatur an der Belagssonde den Oberflächentemperaturen an den Wärmeübertragungsflächen im Kessel entspricht. Die Fluidleitungen ermöglichen jedoch auch die Einstellung eines Temperaturgradienten an der Oberfläche der Sonde, um die Ablagerung und die Belagsqualitäten an der Sonde in Abhängigkeit von der Oberflächentemperatur der Sonde zu untersuchen.

Insbesondere bei unterschiedlichen Oberflächentemperaturen an der Sonde wird vorgeschlagen, dass die Belagssonde Temperaturmesseinrichtungen aufweist. Dies ermöglicht es, die Sondentemperatur einzustellen, zu regeln und beliebig zu variieren.

Die Temperaturüberwachung und insbesondere eine Temperaturregelung führt dazu, dass der Belag bei einer bekannten Oberflächentemperatur des Untergrundes abgelagert wird. Hierzu kann über eine Kühlung der Belagssonde eine bestimmte Oberflächentemperatur eingestellt werden. Außerdem ist es vorteilhaft, wenn die Temperatur des Proberaumes mit dem Tropfengeber gemessen und vorzugweise eingestellt oder sogar geregelt wird, um den Einfluss der Online-Reinigung auf die Rohre und deren Abkühlung zu ermitteln.

Die der Erfindung zugrunde liegende Aufgabe wird auch mit einem gattungsgemäßen Verfahren gelöst, bei dem die Belagssonde aus der Verbrennungsanlage direkt in einen an die Verbrennungsanlage anschließenden auf über 100 °C beheizten Proberaum gezogen wird und dort untersucht wird.

Die Messapparatur und insbesondere das erfindungsgemäße Verfahren ermöglichen es somit, Belagssonden bei Temperaturen- und Gasumgebungen zu untersuchen, die üblicherweise bei der Untersuchung einer Belagssonde nicht vorliegen. Gerade das Überführen der Belagssonde aus der Verbrennungsanlage direkt in den Proberaum, in dem anschließend die Untersuchungen vorgenommen werden, führt zu Untersuchungsergebnissen, die auf die Situation der im Kessel gebildeten Beläge übertragbar sind. Das Verfahren ermöglicht es somit wiederholte Messungen an einer Belagssonde durchzuführen, ohne dass die Belagssonde abkühlt oder Feuchtigkeit zieht. Diese Messergebnisse können dazu verwendet werden, festzulegen, wie und wann im Kessel gebildete Beläge effektiv entfernt werden können.

Ferner wird die Aufgabe mit einer Verbrennungsanlage gelöst, die mit Verbrennungsgasen durchströmbar ist und bei der mindestens eine derartige Messapparatur an einer Außenwand der Verbrennungsanlage positioniert ist, wobei die Belagssonde durch eine Öffnung in der Außenwand in einen von Verbrennungsgasen beströmbaren Innenraum einführbar ist.

Vorteilhaft ist es, wenn die Belagsonde einen Bereich des Innenraums erreichen kann, in dem mindestens ein Wärmetauscher vorgesehen ist.

Eine besondere Ausführungsvariante sieht vor, dass die Verbrennungsanlage mindestens ein Reinigungsgerät für den Innenraum sowie eine Kontrolleinheit umfasst, und die Kontrolleinheit eingerichtet ist, um mit der mindestens einen Messapparatur und dem mindestens einen Reinigungsgerät zusammenzuwirken.

Eine derartige Verbrennungsanlage kann mit einem Verfahren betrieben werden, das die folgenden Verfahrensschritte aufweist:
a) Ermitteln eines Kennwertes für Verbrennungsrückstände in einem Innenraum der Verbrennungsanlage mittels mindestens einer derartigen Messapparatur,
b) Bewerten des Kennwertes in einer Kontrolleinheit im Hinblick auf einen vorgegebenen Grenzwert und
c) Durchführen zumindest eines Reinigungsprozesses im Innenraum oder einer Anpassung des Verbrennungsvorgangs, wenn der Grenzwert erreicht ist.

Bei diesem Verfahren kann bei der Durchführung zumindest des Schrittes a) oder b) eine Temperatur an einem Wärmetauscher berücksichtigt werden.

Ein Ausführungsbeispiel einer Messapparatur ist in der Zeichnung dargestellt und wird im Folgenden näher beschrieben. Es zeigt
- Figur 1: schematisch eine Messapparatur mit in einen Kesselraum geschobener Belagssonde,
- Figur 2: schematisch eine Messapparatur mit aus dem Kesselraum in einen Proberaum gezogener Belagssonde und
- Figur 3: schematisch eine Kesselanlage mit Messapparatur.

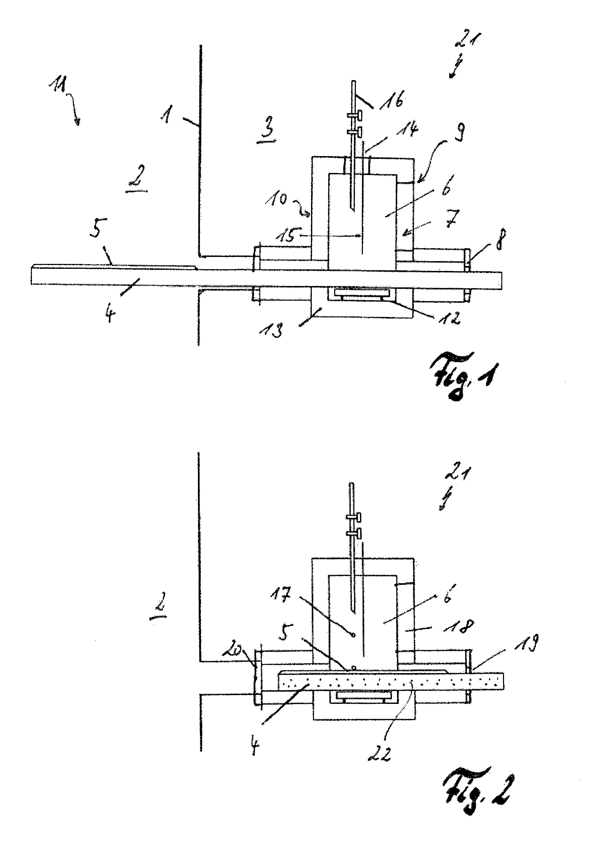

Die Figur 1 zeigt die Kesselwand 1, die einen Kesselraum 2 als Innenraum von einem Außenraum 3 trennt. Eine Belagssonde 4 ist so im Kesselraum 2 angeordnet, dass sich durch die im Kesselraum 2 strömenden Rauchgase auf der Belagssonde 4 eine Belagsschicht 5 niederschlägt. Im Außenraum 3 befindet sich ein Proberaum 6, durch den die Belagssonde 4 geführt ist. Ein an der Wand 7 des Proberaums 6 angeordneter Sondenhalter 8 erlaubt es, die Belagssonde 4 an der Seite 9 des Proberaums 6 in den Proberaum zu schieben und an der gegenüberliegenden Seite 10 aus dem Proberaum 6 heraus in den Kesselraum 2 zu schieben.

Dies ermöglicht es, die Belagssonde 4 in einer Verbrennungsanlage 11 anzuordnen und aus der Verbrennungsanlage 11 direkt in den Proberaum 6 zu ziehen. Eine herausgezogene Sonde 4, die im Proberaum 6 angeordnet ist, zeigt Figur 2.

Der Proberaum 6 ist über eine Heizplatte 12 beheizt und über eine Isolationshülle 13 wärmeisoliert. Ein Messfenster 14 ermöglicht es, ein Thermoelement 15 und einen Tropfengeber 16 in den Proberaum 6 einzuführen. Das Thermoelement 15 misst die Temperatur im Proberaum 6 und der Tropfengeber 16 ist so angeordnet, dass mit ihm dosiert Wassertropfen 17 auf die Belagsschicht 5 der Belagssonde 4 getropft werden können. Ein Sichtfenster 18 ermöglicht es, die Vorgänge im Proberaum 6 von außen zu beobachten.

Ein erster abdichtbarer Belagssondendurchlass 19 dichtet in Form einer Stopfbuchse die Belagssonde 4 zum Proberaum 6 hin ab. Gegenüberliegend ist eine Öffnung 20 als Belagssondendurchlass vorgesehen, der als Schieber den Proberaum 6 gegen den Kesselraum 2 abdichtet. Dies ermöglicht es, die gesamte Messapparatur 21 von der Verbrennungsanlage 11 zu lösen, ohne die Belagssonde 4 aus dem Proberaum 6 zu entfernen.

Damit die Belagssonde 4 an ihrer Oberfläche eine etwa den Wärmeübertragungsflächen im Kesselraum 2 entsprechende Temperatur aufweist, sind in der Belagssonde Fluidleitungen 22 zur Beeinflussung der Sondentemperatur vorgesehen.

In der Praxis wird zunächst mit diesen Fluidleitungen ein erhitzter Gas- oder Dampfstrom durch die Belagssonde geleitet, um die Belagssonde auf eine Temperatur von mehreren Hundert Grad Celsius zu erhitzen. Die derart erhitzte Belagssonde 4 wird mehrere Stunden, Tage oder Wochen im Kesselraum 2 den Rauchgasen ausgesetzt, sodass sich eine Belagsschicht 5 auf der Belagssonde 4 ablagert. Anschließend wird die Belagssonde 4 mit der Belagsschicht 5 vorsichtig durch den Belagssondendurchlass 20 in den Proberaum 6 überführt, sodass im ebenfalls auf mehrere Hundert Grad Celsius beheizten Proberaum die Belagsschicht 5 an der Belagssonde 4 untersucht werden kann.

Nach den Untersuchungen kann die Belagssonde 4 oder eine neue Belagssonde durch den Proberaum 6 in den Kesselraum 2 geschoben werden.

Selbst wenn die Temperaturen im Proberaum weit niedriger liegen sollten als im Kesselraum, sorgt bereits die Überführung der Sonde aus dem Kesselraum direkt in den Proberaum dafür, dass der Belag aus der Atmosphäre keine Feuchtigkeit zieht und sich nicht wesentlich verändert.

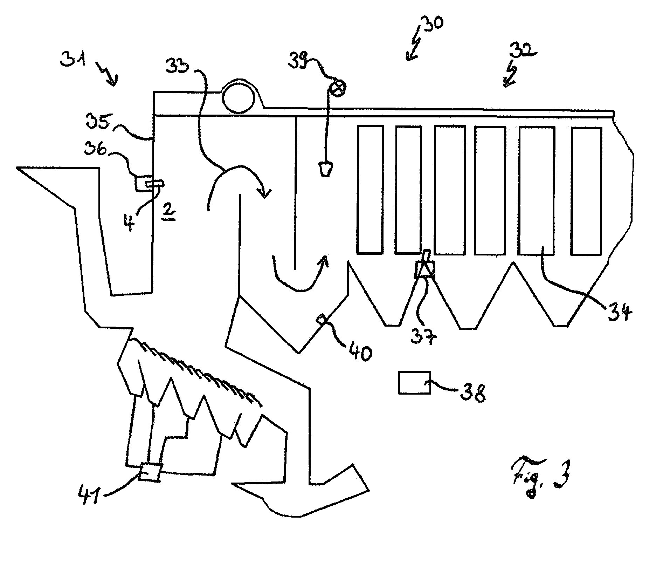

Die Figur 3 zeigt eine Verbrennungsanlage 30 mit einem Feuerungsraum 31 und einen nachgelagerten so genannten Konvektionsteil 32, die beide im Betrieb von Verbrennungsgasen 33 durchströmt werden. Im Feuerungsraum 31 können innen an der Wand Rohrschlangen (nicht gezeigt) vorgesehen sein, die einen Wärmetauscher bilden. Im Bereich des Feuerungsraums 31 und/oder des Konvektionsteils 32 können zudem (auch) als Wärmetauscher 34 im Innenraum hängende Rohrschlangenpakete positioniert sein, die von den Verbrennungsgasen 33 durch- bzw. umströmt werden und einen Wärmetauscher bilden.

Im Ausführungsbeispiel ist an der Kesselwand 35 des Feuerungsraumes 31 eine Messapparatur 36 und am Konvektionsteil 32 eine Messapparatur 37 an der Außenwand der Verbrennungsanlage 30 montiert. Im Bereich der Messapparaturen 36, 37 weist die Außenwand eine (ggf. verschließbare) Öffnung 20 auf, durch die eine Belagssonde 4 zeitweise in den Innenraum eingeführt werden kann. Dabei kann die Belagssonde 4 bevorzugt einen Bereich im Innenraum erreichen, in dem ein Wärmetauscher 34 vorgesehen ist.

Damit wird es möglich, mit einer Messapparatur 36, 37 einen charakteristischen Kennwert für die Beläge an einem Wärmetauscher 34 zu generieren. Der ermittelte Kennwert kann dann von einer Kontrolleinheit 38 ausgewertet werden. Ergibt sich aus dieser Bewertung, dass ein vorgegebener und/oder an den aktuellen Betrieb der Verbrennungsanlage 30 angepasster Grenzwert (betreffend z.B. die Festigkeit des Belags, die Belagdicke, die Art des Belags, ... etc.) erreicht bzw. überschritten wird, kann ein Reinigungsprozess und/oder eine Anpassung des Verbrennungsprozesses ausgeführt werden.

Die Kontrolleinheit 38 ist dazu eingerichtet, mit den Messapparaturen 36, 37, dem Temperaturfühler 40, dem Reinigungsgerät 39 und/oder Stellmitteln 41 für die Verbrennungsanlage, wie Brenner oder Luftklappen, zusammenzuwirken. Somit ist es möglich, die Beläge auf den Wärmetauschern 34 zu einem günstigen Zeitpunkt abzureinigen und/oder die Feuerung so zu beeinflussen, dass die Belagsbildung an den Wärmetauschern 34 günstig beeinflusst wird. Unterstützend kann eine Temperatur an oder in einem Wärmetauscher 34 erfasst oder bestimmt werden, so dass beispielsweise der Zeitpunkt zum Start des Schrittes der Bewertung des Kennwertes bzw. der Festlegung des Grenzwerts unter Berücksichtigung dieser Temperatur erfolgen kann.

Als Reinigungsgerät 39 kommen beispielsweise an der Außenwand montierte und/oder durch die Außenwand einfahrbare Wasser- und/oder Dampfabgabegeräte in Betracht, insbesondere so genannte Wasserlanzenbläser, Rußbläser, Schlauchsprinkler, etc. Zudem können auch Klopfer, (Druck-) Luftreiniger und/oder Explosionsgeneratoren Verwendung finden.

## Patentansprüche

1. Messapparatur mit einer Belagssonde (4) und einem Sondenhalter, ***dadurch gekennzeichnet, dass*** der Sondenhalter (8) an einer Wand (7) eines Proberaumes (6) angeordnet ist und die Belagssonde (4) an einer Seite (9) in den Proberaum (6) und an einer gegenüberliegenden Seite (10) aus dem Proberaum (6) heraus verschiebbar ist.

2. Messapparatur nach Anspruch 1, ***dadurch gekennzeichnet, dass*** der Proberaum (6) beheizt ist.

3. Messapparatur nach Anspruch 1 oder 2, ***dadurch gekennzeichnet, dass*** der Proberaum (6) wärmeisoliert ist.

4. Messapparatur nach einem der vorhergehenden Ansprüche, ***dadurch gekennzeichnet, dass*** der Proberaum (6) ein Messfenster (14) aufweist.

5. Messapparatur nach einem der vorhergehenden Ansprüche, ***dadurch gekennzeichnet, dass*** der Proberaum (6) ein Sichtfenster (18) aufweist.

6. Messapparatur nach einem der vorhergehenden Ansprüche, ***dadurch gekennzeichnet, dass*** der Proberaum (6) einen Tropfengeber (16) aufweist.

7. Messapparatur nach einem der vorhergehenden Ansprüche, ***dadurch gekennzeichnet, dass*** der Proberaum (6) zwei gegenüberliegende abdichtbare Belagssondendurchlässe (19, 20) aufweist.

8. Messapparatur nach einem der vorhergehenden Ansprüche, ***dadurch gekennzeichnet, dass*** die Belagssonde (4) Fluidleitungen (22) zur Beeinflussung der Sondentemperatur aufweist.

9. Messapparatur nach einem der vorhergehenden Ansprüche, ***dadurch gekennzeichnet, dass*** die Belagssonde (4) Temperaturmesseinrichtungen aufweist.

10. Verfahren zum Untersuchen von Belägen auf einer Belagssonde (4), bei dem die Belagssonde (4) in einer Verbrennungsanlage (11) angeordnet wird, ***dadurch gekennzeichnet, dass*** die Belagssonde (4) aus der Verbrennungsanlage (11) direkt in einen an die Verbrennungsanlage (11) anschließenden auf über 100 °C beheizten Proberaum (6) gezogen wird und dort untersucht wird.

11. Verbrennungsanlage (30), die mit Verbrennungsgasen (33) durchströmbar ist, ***dadurch gekennzeichnet, dass*** mindestens eine Messapparatur (36, 37) nach einem der Ansprüche 1 bis 9 an einer Außenwand (35) der Verbrennungsanlage (30) positioniert ist und die Belagssonde (4) durch eine Öffnung in der Außenwand (35) in einen von Verbrennungsgasen (33) beströmbaren Innenraum (2) einführbar ist.

12. Verbrennungsanlage (30) nach Anspruch 11, ***dadurch gekennzeichnet, dass*** die Belagssonde (4) einen Bereich des Innenraums (2) erreichen kann, in dem mindestens ein Wärmetauscher (34) vorgesehen ist.

13. Verbrennungsanlage (11) nach Anspruch 11 oder 12, ***dadurch gekennzeichnet, dass*** die Verbrennungsanlage (30) mindestens ein Reinigungsgerät (39) für den Innenraum (2) sowie eine Kontrolleinheit (38) umfasst, und die Kontrolleinheit (38) eingerichtet ist, um mit der mindestens einen Messapparatur (36, 37) und dem mindestens einen Reinigungsgerät (39) zusammenzuwirken.

14. Verfahren zum Betrieb einer Verbrennungsanlage (30) nach einem der Ansprüche 11 bis 13, umfassend zumindest die folgenden Schritte:
a) Ermitteln eines Kennwertes für Verbrennungsrückstände in einem Innenraum (2) der Verbrennungsanlage (30) mittels mindestens einer Messapparatur (36, 37) gemäß einem der Ansprüche 1 bis 9,
b) Bewerten des Kennwertes in einer Kontrolleinheit (38) im Hinblick auf einen vorgegebenen Grenzwert,
c) Durchführen zumindest eines Reinigungsprozesses im Innenraum (2) oder einer Anpassung des Verbrennungsvorgangs, wenn der Grenzwert erreicht ist.

15. Verfahren nach Anspruch 14, ***dadurch gekennzeichnet, dass*** bei der Durchführung zumindest des Schrittes a) oder b) eine Temperatur an einem Wärmetauscher (34) berücksichtigt wird.
